# FASCICULE DE BREVET EUROPEEN

(11) **EP 3 950 001 B1**
(45) Date de publication et mention de la délivrance du brevet: **15.04.2026**
(21) Numéro de dépôt: 21188085.1
(22) Date de dépôt: 27.07.2021
(51) Int. Cl.: A61K 47/10, A61K 47/14, A61K 47/34, A61K 8/02, A61K 8/86, A61Q 19/00

(54) **UTILISATION COMME EXCIPIENT, D'UN MELANGE DE MACROGOLGLYCERIDE LAURIQUE ET DE POLYETHYLENE GLYCOL**
VERWENDUNG EINES GEMISCHS AUS LAURIN-MACROGOLGLYCERID UND POLYETHYLENGLYKOL ALS HILFSSTOFF
USE OF A MIXTURE OF LAUROYL MACROGOLGLYCERIDE AND POLYETHYLENE GLYCOL AS A CARRIER

(30) Priorité: 05.08.2020 FR 2008312
(43) Date de publication de la demande: 09.02.2022
(73) Titulaire: GATTEFOSSE SAS, 69804 Saint-Priest Cedex (FR)
(72) Inventeur: FOREST, Amandine, 69008 LYON (FR); RODIER, Jean-David, 69100 VILLEURBANNE (FR)
(74) Mandataire: Ipsilon

(56) Documents cités:
- US-A1- 2016 120 964
- "Bull. Korean Chem. Soc.", vol. 38, 6 November 2007, WILEY - V C H VERLAG GMBH & CO. KGAA, DE, ISSN: 0931-7597, article VASANTHAVADA MADHAV ET AL: "Lipid-Based Self-Emulsifying Solid Dispersions", pages: 149 - 183, XP055797152, DOI: 10.1002/chin.200745270

## Description

La présente invention se rapporte à un excipient à usage pharmaceutique ou cosmétique, à base de macrogolglycéride laurique et de polyéthylène glycol (PEG ou macrogol). La description concerne également un procédé de fabrication d'une composition pharmaceutique ou cosmétique comprenant un tel excipient.

Le Gélucire^{®}44/14, identifié dans la Pharmacopée européenne sous la dénomination "Macrogolglycéride laurique", est un excipient connu, commercialisé par le Demandeur depuis de nombreuses années. Ce produit est constitué d'un mélange de mono-, di- et triesters de glycérol et de mono- et diesters de PEG avec des acides gras saturés, dont le nombre de carbone est compris entre 8 et 18. Le mélange contient également une faible proportion de PEG et de glycérol libre. De tels mélanges peuvent être obtenus par réaction d'alcoolyse d'une huile végétale hydrogénée, par exemple l'huile de coprah hydrogénée par le polyéthylène glycol. Cette huile constituée elle-même de triglycérides contenant les acides gras décrits ci-avant. Un tel excipient peut également être obtenu par estérification de glycérol et de PEG avec les acides gras précédemment décrits ou encore un mélange d'esters de glycérol et de condensat d'oxyde d'éthylène avec lesdits acides gras. Le Gélucire^{®} 44/14 présente un point de fusion d'environ 44°C et un nombre HLB de 14.

Comme mentionné ci-dessus, le Gélucire^{®}44/14 est largement utilisé comme excipient dans les compositions pharmaceutiques, ou même parfois cosmétiques, pour ses propriétés d'amélioration de la solubilité des actifs notamment et leur biodisponibilité.

Le document EP1972336 décrit ainsi un procédé de fabrication de micro-pellets, dont le diamètre est de l'ordre du micromètre, en particulier inférieur à 500 micromètres. Ces micro-pellets comprennent du kétoprofène dispersé dans une matrice de Gélucire^{®}44/14 ou de PEG 4000 ou de Lutrol F68, chacun des excipients étant montré comme ayant un pouvoir de dissolution de l'actif.

Le document US2016120964 décrit des compositions pharmaceutiques obtenues par granulation par voie humide constituées de pancréatine de porc, utilisé en tant que principe actif, de Gélucire^{®}44/14 et de PEG 4000.

Le document US2003220391 décrit des capsules contenant un dérivé de taxane, du Gélucire^{®}44/14 et du PEG 1450. En pratique, le Gélucire^{®} 44/14 et le PEG sont introduits séparément dans une cuve chauffée. Une fois le mélange à l'état liquide, on introduit l'actif dans la cuve et on remplit enfin les capsules avec le mélange obtenu.

Le Gélucire^{®}44/14 est un produit pâteux qui se présente en bloc hétérogène. Il ne peut pas être conditionné sous forme divisée, c'est-à-dire par exemple sous forme de granulés, de pastilles ou d'écailles.

Manipuler le Gélucire^{®}44/14 requiert en effet de fondre l'ensemble de l'échantillon avant sa mise en œuvre, même si la quantité souhaitée pour la formulation est inférieure à la quantité de l'échantillon. Cette opération est nécessaire pour s'assurer de l'homogénéité du prélèvement. Elle nécessite d'utiliser une étuve, et induit donc la manipulation d'un produit liquide chaud, ce qui constitue un premier inconvénient. En outre, la manipulation de la matière peut entrainer des pertes en fonction de la quantité finalement utilisée, ce qui constitue un second inconvénient.

Le Gélucire^{®}44/14 est donc un produit nécessitant une certaine manutention de même qu'une maîtrise de la quantité à utiliser, qu'il convient d'améliorer.

Le document XP 55797152 décrit séparément l'utilisation de Gélucire^{®}44/14 et de PEG en tant qu'excipient pharmaceutique pour améliorer la solubilité et la biodisponibilité des actifs pharmaceutiques.

Un but de l'invention est ainsi celui de rendre plus facile et plus rapide la manipulation du Gélucire^{®}44/14 lors de la préparation de formulations pharmaceutiques ou cosmétiques.

Un autre but de l'invention est de rendre possible le prélèvement de Gélucire^{®} 44/14 en quantité inférieure à celle contenue dans un emballage industriel, tout en assurant le prélèvement d'une composition homogène.

Le Demandeur a constaté que de manière tout à fait surprenante, la manipulation par le formulateur du Gélucire^{®}44/14 à température ambiante est facilitée lorsqu'il est associé à du PEG. Ce PEG ayant la caractéristique d'être solide à température ambiante. Il s'avère en effet possible d'obtenir une forme solide divisible de Gélucire^{®}44/14. Le formulateur dispose ainsi de Gélucire^{®}44/14 sous forme solide individualisée, qu'il peut utiliser dans la quantité exactement souhaitée.

En d'autres termes, l'invention a pour objet l'utilisation comme excipient dans une formulation pharmaceutique ou cosmétique, d'une composition solide à température ambiante, sous forme de particules individualisées, ladite composition contenant uniquement:
- macrogolglycéride laurique (qui est l'équivalent en nomenclature chimique du nom commercial Gélucire^{®}44/14),
- polyéthylène glycol de masse molaire comprise entre 4000 g/mol et 8000 g/mol.

Par conséquent, de par son utilisation comme excipient, la composition solide de l'invention ne contient pas de principe actif thérapeutique.

Le mélange du macrogolglycéride laurique avec un polyéthylène glycol n'engendre pas de réaction chimique. Aucune nouvelle entité chimique n'est créée lors du mélange du Gélucire^{®}44/14 avec du PEG, ce qui est vérifié par l'expérience.

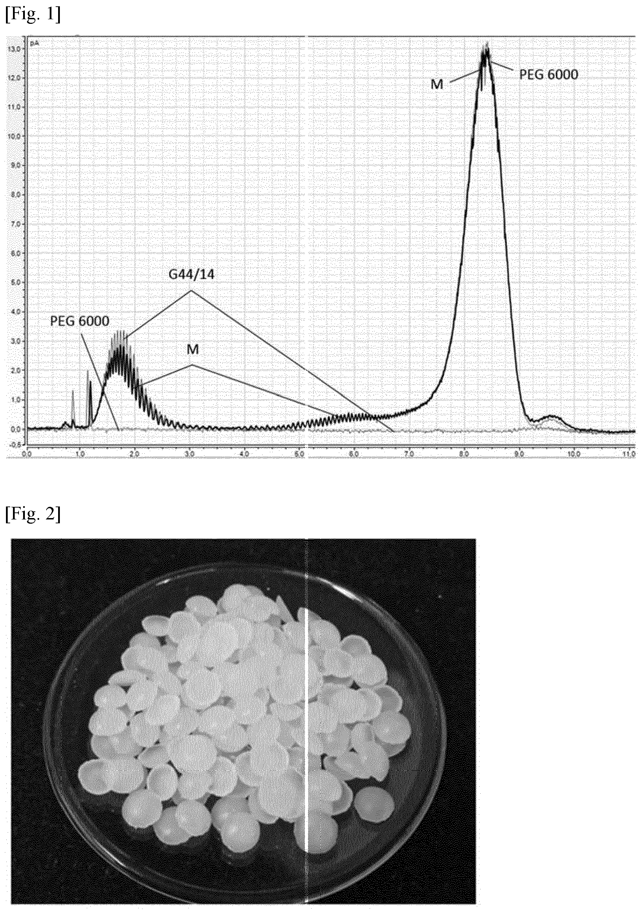

La figure 1 est un graphe qui permet de comparer un mélange Gélucire^{®}44/14 / PEG 6000 au ratio massique de 65/35 avec la distribution du PEG 6000 et du Gélucire^{®}44/14 pris isolément. Ces résultats sont obtenus en HPLC-CAD (Charged Aerosol Detector en anglais, ou détecteur d'aérosol chargé en français). Sur la figure 1, le Gélucire^{®}44/14 est noté G44/14, et le mélange est Gélucire^{®}44/14 / PEG 6000 est noté M. On constate qu'aucune entité chimique autre que le Gélucire^{®}44/14 et le PEG 6000 n'est présente sur ce graphe confirmant ainsi l'absence de réaction entre le PEG et le Gélucire^{®}.

Comme déjà dit, le Gélucire^{®}44/14 contient des mono, di et triglycérides d'acides gras saturés de C8 à C18, et des mono- et diesters de PEG 1500 d'acides gras de C8 à C18. Il peut éventuellement contenir du glycérol libre et du PEG 1500 libre. Néanmoins, il est clair que le PEG mentionné dans le paragraphe précédent est ajouté au Gélucire^{®}44/14, c'est-à-dire éventuellement à la fraction de PEG présente initialement dans le Gélucire^{®}44/14. En d'autres termes, le PEG ne provient pas du PEG 1500 libre présent dans le Gélucire^{®} 44/14.

Dans un mode de réalisation avantageux, la composition contient uniquement ces deux constituants, Gélucire^{®}44/14 et PEG solide à température ambiante.

Le Demandeur a constaté qu'on obtenait des résultats particulièrement avantageux lorsque la masse molaire du PEG est comprise entre 4000 g/mol et 8000 g/mol, avantageusement entre 4000 g/mol et 6000 g/mol.

Avantageusement, le ratio massique Gélucire^{®}44/14 / polyéthylène glycol est compris entre 40/60 et 70/30.

Le polyéthylène glycol (PEG) est un excipient à point de fusion élevé, compris entre environ 53°C et environ 59 °C pour le PEG 4000, et entre environ 55°C et 62°C pour le PEG 8000. L'ajout de ce PEG à du Gélucire^{®}44/14 permet d'obtenir un mélange solide et non pâteux. Contrairement au Gélucire^{®}44/14 seul, le mélange Gélucire^{®}44/14 et PEG est alors divisible, c'est-à-dire qu'il est possible de fabriquer des particules individualisées comme des pastilles plutôt que de former des blocs de Gélucire^{®}.

La composition peut comprendre un seul PEG ou un mélange d'au moins deux PEG différents, c'est-à-dire de masses molaires différentes, dans des quantités identiques ou différentes. Dans le cas d'un mélange de PEG, les PEG ont tous avantageusement une masse molaire comprise entre 4000 g/mol et 8000 g/mol, et de préférence comprise entre 4000 g/mol et 6000 g/mol.

La masse molaire du PEG et le ratio massique Gélucire^{®}44/14 / polyéthylène glycol qui sont décrits sont relatifs au PEG qui est rajouté au Gélucire^{®}44/14 contenant initialement une fraction de PEG 1500.

Lorsque la composition solide comprend plusieurs PEG différents, le ratio massique décrit précédemment est calculé par rapport à la masse totale des PEG. Par exemple, lorsque la pastille solide comprend deux PEG différents, notés PEG 1 et PEG 2, le ratio massique est le suivant : masse Gélucire^{®}44/14 / (masse PEG 1 + masse PEG 2). Ce ratio est compris entre 40/60 et 70/30.

En pratique, les particules individualisées ont une taille d'au moins 1 mm. Par l'expression « particules individualisées », on désigne des particules, de préférence des pastilles, dont la taille est comprise entre 1 mm et 15 mm et avantageusement entre 5 mm et 10 mm.

Le Demandeur a constaté, qu'il était possible d'obtenir des pastilles non collantes et intègres, en particulier dans les fourchettes de masses molaires et ratios massiques précités.

De telles pastilles sont obtenues par une étape de pastillage bien connue de l'homme du métier. Ce pastillage peut s'effectuer manuellement à l'aide d'une pipette ou industriellement à l'aide d'une pastilleuse industrielle.

Une pastille est un objet possédant une face sensiblement plate et une face bombée. La taille caractéristique de la pastille solide est de préférence supérieure ou égale à 1 mm, et de manière avantageusement préférée supérieure ou égale à 5 mm. La taille caractéristique correspond à la plus grande longueur de la face plate de la pastille. Lorsque la pastille est sensiblement ronde, sous forme de disque, la taille caractéristique correspond au diamètre de la face plate. Lorsque la pastille présente sur sa face plate une forme oblongue (ellipse), la taille caractéristique correspond au diamètre maximal de la face plate soit le grand axe de l'ellipse. Sous forme de pastille, le mélange Gélucire^{®}44/14 et PEG est aisément manipulable par le formulateur industriel, et en toute sécurité, contrairement au Gélucire^{®}44/14 seul sous forme d'un bloc pâteux à faire fondre entièrement. Le formulateur industriel peut facilement peser et manipuler la quantité de pastilles nécessaire à la préparation du mélange actif, sans qu'il ne faille faire fondre l'ensemble du bloc de Gélucire^{®}44/14 et manipuler un liquide à haute température. Le Demandeur a démontré que l'utilisation, en combinaison avec du Gélucire^{®}44/14, d'un PEG dont la masse molaire est avantageusement comprise entre 4000 g/mol et 8000 g/mol, de manière davantage préférée entre 4000 g/mol et 6000 g/mol, et à un ratio massique Gélucire^{®}44/14 / polyéthylène glycol avantageusement compris entre 40/60 et 70/30, améliorait les propriétés de dissolution de l'actif seul.

Selon l'invention, le procédé de fabrication de la composition comprend les étapes suivantes :
- chauffage du Gélucire^{®}44/14 et du polyéthylène glycol (PEG) à une température T1 de manière à obtenir un mélange liquide de Gélucire^{®}44/14 et de polyéthylène glycol,
- refroidissement du mélange de macrogolglycéride laurique et de polyéthylène glycol à une température T2 inférieure à la température T1, tout en conservant le mélange liquide,
- division du mélange refroidi, avantageusement sous forme de gouttes et gouttelettes,
- refroidissement du mélange divisé, avantageusement sous forme de gouttes et gouttelettes, à une température T3 inférieure à la température T2 pour obtenir des particules solides individualisées.

La température T3 est avantageusement la température ambiante, comprise entre 20 et 25 °C.

De préférence, avant l'étape de refroidissement, le mélange Gélucire^{®}44/14 et PEG est agité pendant au moins 15 minutes à la température T1.

Le Gélucire^{®}44/14 et le PEG sont de préférence mélangés l'un avec l'autre avant l'étape de chauffage, puis le mélange de Gélucire^{®}44/14 et de polyéthylène glycol est chauffé à la température T1.

On choisira avantageusement une température T1 supérieure ou égale à 80°C, afin de s'assurer que la totalité du Gélucire^{®}44/14 et du PEG a fondu, et se trouve à l'état liquide.

En outre, on choisira avantageusement une température T2 supérieure ou égale à 45°C, de préférence supérieure ou égale à 55°C, et inférieure ou égale à 60°C, afin que le refroidissement soit relativement doux et que la quantité de chaleur à évacuer ne soit pas trop élevée, pour s'assurer de ne pas dégrader le Gélucire^{®}44/14 et le PEG. Une température de 55°C correspond à environ 10°C au-dessus du point d'inflexion du thermo-rhéogramme obtenu pendant l'étape de refroidissement. En effet, le Gélucire^{®}44/14 présente un large endotherme allant de 10°C à 45°C, avec une température de fusion initiale d'environ 38°C et une température de fusion maximale d'environ 43°C.

Le mélange Gélucire^{®}44/14 / PEG 6000 dans les proportions massiques 65 / 35 a un point de goutte déterminé à 58.3°C. Le mélange Gélucire^{®}44/14 / PEG 6000 selon les proportions massiques 60 / 40 a un point de goutte déterminé à 58.6°C. Le refroidissement du mélange de Gélucire^{®}44/14 et PEG est de préférence réalisé en déposant des gouttes dudit mélange sur une surface dont la température comprise entre 4°C et 8°C.

La description concerne également un procédé de fabrication d'une composition pharmaceutique selon lequel :
- on fond les particules individualisées d'excipient telle que décrites précédemment pour obtenir une masse liquide,
- on introduit l'actif dans la masse ainsi liquéfiée,
- on transforme le mélange obtenu dans la forme galénique souhaitée.

Indépendamment de la nature de l'excipient qui fait l'objet de l'invention, l'ensemble de ces étapes est bien connu de l'homme du métier.

Ainsi, le choix des excipients autres que celui faisant l'objet de l'invention pour obtenir ladite composition pharmaceutique, de même que les différentes opérations de transformation (comprimés, gélules etc...) sont à la portée de l'homme du métier et ne nécessitent pas d'être d'avantage explicités.

L'invention et les avantages qui en découlent ressortiront bien des exemples de réalisation qui suivent, à l'appui des figures jointes, dans lesquelles :
[Fig. 1] (décrite précédemment) est un graphe HPLC-CAD (Charged Aerosol Detector en anglais, ou détecteur d'aérosol chargé en français) qui illustre les distributions du Gélucire^{®}44/14 seul, du PEG 6000 seul, et d'un mélange (noté M) Gélucire^{®}44/14 / PEG 6000 au ratio massique de 65/35 conforme à l'invention;
[Fig. 2] est une photographie de pastilles solides conformes à l'invention ;
[Fig. 3a] est un graphe illustrant la libération du piroxicam en fonction du temps, pour plusieurs formulations ;
[Fig. 3b] est un graphe illustrant la libération du piroxicam en fonction du temps, pour plusieurs formulations additionnelles à la figure 3a ;
[Fig. 4] est un graphe illustrant la libération de la terfénadine en fonction du temps, pour plusieurs formulations ;
[Fig. 5] est un graphe illustrant la libération de l'ibuprofène en fonction du temps, pour plusieurs formulations.

### Exemple 1 : préparation de l'excipient selon l'invention

Différentes formulations de pastilles solides obtenues selon le procédé décrit précédemment sont listées dans le tableau I ci-dessous.

### [Table I]

Les compositions comprenant du PEG 1500 ne font pas partie de l'invention et sont des exemples comparatifs.

**Tableau I**

| **Ratio** | **Température mélange (°C)** | **Température de refroidissement (°C)** |
|---|---|---|
| **Gelucire^{®} 44/14 / PEG** | | |
| Gelucire^{®} 44/14 / PEG 1500 = 10 / 90 | 48 - 51 | 5,0 - 5,5 |
| Gelucire^{®} 44/14 / PEG 4000 = 40 / 60 | 55 - 58 | 4,5 - 6,5 |
| Gelucire^{®} 44/14 / PEG 4000 = 55 / 45 | 54 - 55 | 4,8 - 6,0 |
| Gelucire^{®} 44/14 / PEG 4000 = 60 / 40 | 51 - 54 | 4,0 - 5,0 |
| Gelucire^{®} 44/14 / PEG 4000 = 65 / 35 | 54 - 57 | 5,0 - 6,0 |
| Gelucire^{®} 44/14 / PEG 4000 = 70 / 30 | 55 | 6,5 |
| Gelucire^{®} 44/14 / PEG 6000 = 60 / 40 | 57 - 60 | 5,5 à 8,5 |
| Gelucire^{®} 44/14 / PEG 6000 = 65 / 35 | 56 - 57 | 4,5 à 6,5 |
| Gelucire^{®} 44/14 / PEG 6000 = 70 / 30 | 60 | 5,5 à 7,5 |
| Gelucire^{®} 44/14 / PEG 8000 = 80 / 20 | 55 | 6,0 |
| Gelucire^{®} 44/14 / PEG 6000 + PEG 1500 = 50 / 20+30 | 53 - 57 | 5,5 - 6,5 |
| Gelucire^{®} 44/14 / PEG 6000 + PEG 1500 = 50 / 30+20 | 53 - 55 | 5,5 - 7,5 |
| Gelucire^{®} 44/14 / PEG 6000 + PEG 1500 = 65 / 30+5 | 54 - 59 | 7,2 - 8,0 |
| Gelucire^{®} 44/14 / PEG 6000 + PEG 4000 = 65 / 25+10 | 55 | 4,0 |
| Gelucire^{®} 44/14 / PEG 6000 + PEG 4000 = 65 / 30+5 | 57 - 59 | 6,0 |
| Gelucire^{®} 44/14 / PEG 6000 + PEG 8000 = 65 / 30+5 | 51 - 58 | 4,0 - 6,2 |

Les pastilles solides obtenues avec un mélange Gélucire^{®}44/14 / PEG 6000 selon les proportions massiques suivantes : 65/35 sont illustrées sur la figure 2. Elles sont opaques et ont une forme de disque sur la face plate.

Le mélange Gélucire^{®}44/14 / PEG 6000 dans les proportions massiques 65 / 35 a un point de goutte déterminé à 58.3°C. Le mélange Gélucire^{®}44/14 / PEG 6000 selon les proportions massiques 60 / 40 a un point de goutte déterminé à 58.6°C.

### Exemple 2 : impact du PEG sur la dissolution des actifs

### Exemple 2.1 : piroxicam

Un actif, le piroxicam, a été formulé avec du Gélucire^{®}44/14. Le mélange obtenu a été coulé dans une gélule.

La solubilité du piroxicam dans l'eau, à 25°C, est de 23 mg/L.

On obtient les gélules contenant la formulation suivante : 20 mg de piroxicam, et 660 mg de Gélucire^{®}44/14.

On compare les performances de dissolution du piroxicam avec différentes formulations de pastilles solides obtenues de la même manière que précédemment, à la différence que le Gélucire^{®}44/14 est préalablement préparé en présence de PEG comme expliqué précédemment.

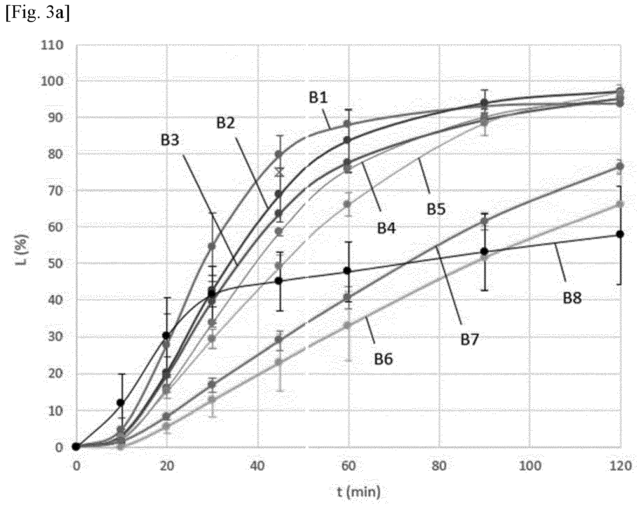

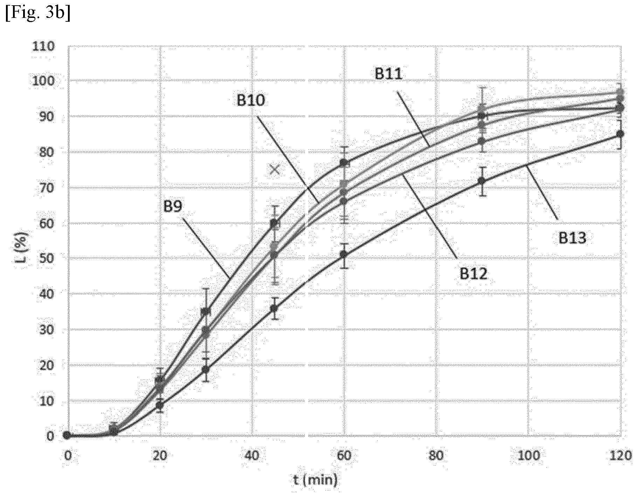

On obtient alors les graphes des figures 3a et 3b, illustrant la libération L (%) de l'actif en fonction du temps t en minutes, sur lesquels :
Figure 3a
   - la courbe B1 (invention) correspond à un mélange piroxicam / Gélucire^{®}44/14 / PEG 4000, dont le ratio massique Gélucire^{®}44/14 / PEG 4000 est de 55 / 45 ;
   - la courbe B2 (invention) correspond à un mélange piroxicam / Gélucire^{®}44/14 / PEG 6000, dont le ratio massique Gélucire^{®}44/14 / PEG 6000 est de 65 / 35 ;
   - la courbe B3 (invention) correspond à un mélange piroxicam / Gélucire^{®}44/14 / PEG 4000, dont le ratio massique Gélucire^{®}44/14 / PEG 4000 est de 60 / 40 ;
   - la courbe B4 (invention) correspond à un mélange piroxicam / Gélucire^{®}44/14 / PEG 6000, dont le ratio massique Gélucire^{®}44/14 / PEG 6000 est de 60 / 40 ;
   - la courbe B5 (invention) correspond à un mélange piroxicam / Gélucire^{®}44/14 / PEG 6000, dont le ratio massique Gélucire^{®}44/14 / PEG 6000 est de 70 / 30 ;
   - la courbe B6 (invention) correspond à un mélange piroxicam / Gélucire^{®}44/14 / PEG 4000, dont le ratio massique Gélucire^{®}44/14 / PEG 4000 est de 40 / 60 ;
   - la courbe B7 (témoin 1) correspond à un mélange piroxicam / PEG 6000 ;
   - la courbe B8 (témoin 2) correspond à du piroxicam seul.
Figure 3b
   - la courbe B9 (invention) correspond à un mélange piroxicam / Gélucire^{®}44/14 / PEG 6000 + PEG 1500, dont le ratio massique Gélucire^{®}44/14 / PEG 1 + PEG 2 est de 50 / 20+30 ;
   - la courbe B10 (invention) correspond à un mélange piroxicam / Gélucire^{®}44/14 / PEG 6000 + PEG 4000, dont le ratio massique Gélucire^{®}44/14 / PEG 1 + PEG 2 est de 65 / 30+5 ;
   - la courbe B11 (invention) correspond à un mélange piroxicam / Gélucire^{®}44/14 / PEG 6000 + PEG 8000, dont le ratio massique Gélucire^{®}44/14 / PEG 1 + PEG 2 est de 65 / 30+5 ;
   - la courbe B12 (invention) correspond à un mélange piroxicam / Gélucire^{®}44/14 / PEG 6000 + PEG 4000, dont le ratio massique Gélucire^{®}44/14 / PEG 1 + PEG 2 est de 65 / 25+10 ;
   - la courbe B13 (invention) correspond à un mélange piroxicam / Gélucire^{®}44/14 / PEG 6000 + PEG 1500, dont le ratio massique Gélucire^{®}44/14 / PEG 1 + PEG 2 est de 65 / 30+5.

On constate que tous les mélanges piroxicam / Gélucire^{®}44/14 / PEG (courbes B1 à B6 et B9 à B13) conduisent à une libération du piroxicam supérieure à celle de l'actif seul (témoin 2, courbe B8).

Les mélanges comprenant du PEG (courbes B1 à B5) conduisent à une libération supérieure à 95% après 2 heures, ce qui est très satisfaisant, hormis pour le mélange piroxicam / Gélucire^{®}44/14 / PEG 4000 dont le ratio massique Gélucire^{®}44/14 / PEG 4000 est de 40 / 60 (courbe B6).

Les mélanges comprenant une association de deux PEG (courbes B9 à B12) conduisent à une libération supérieure à 90% après 2 heures, ce qui est satisfaisant, hormis pour le mélange piroxicam / Gélucire^{®}44/14 / PEG 6000 + PEG 1500 dont le ratio massique Gélucire^{®}44/14 / PEG 6000 + PEG 1500 est de 65 / 30 + 5 (courbe B13).

Par ailleurs, le PEG (témoin 1, courbe B7) seul est beaucoup moins performant qu'en mélange avec le Gélucire^{®}44/14.

### Exemple 2.2 : terfénadine

Un autre actif, la terfénadine, a été formulé avec le Gélucire^{®}44/14. Le mélange a été coulé en gélule, de manière similaire à l'exemple 1 précédent avec le piroxicam.

La solubilité de la terfénadine dans l'eau, à 25°C, est de 250 mg/L.

La dose est de 60 mg, pour un total de 680 mg de formulation.

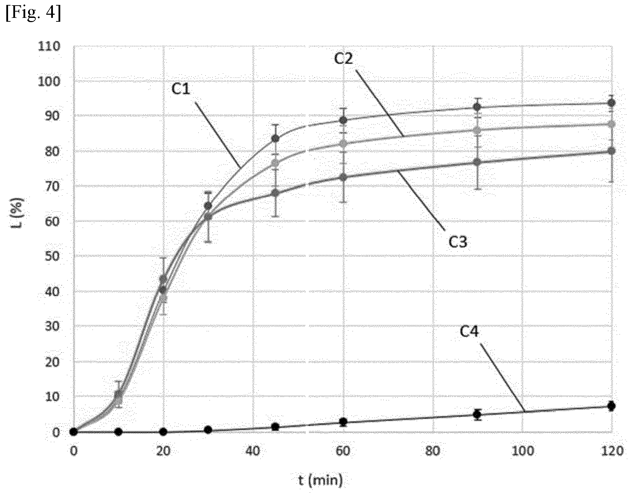

On obtient alors le graphe de la figure 4, illustrant la libération L (%) de l'actif en fonction du temps t en minutes, sur lequel :
- la courbe C1 (invention) correspond à un mélange terfénadine / Gélucire^{®}44/14 / PEG 6000, dont le ratio massique Gélucire^{®}44/14 / PEG 6000 est de 65 / 35 ;
- la courbe C2 (invention) correspond à un mélange terfénadine / Gélucire^{®}44/14 / PEG 6000, dont le ratio massique Gélucire^{®}44/14 / PEG 6000 est de 60 / 40 ;
- la courbe C3 (témoin 1) correspond à un mélange terfénadine / PEG 6000 ;
- la courbe C4 (témoin 2) correspond à de la terfénadine seule.

On constate là aussi que les mélanges terfénadine / Gélucire^{®}44/14 / PEG (courbes C1 et C2) conduisent à une libération d'environ 90% après 90 minutes, ce qui est très satisfaisant. Cette libération est bien supérieure à celle de l'actif seul qui reste inférieure à 10% après 120 minutes (témoin 2, courbe C4).

Par ailleurs, le PEG seul (témoin 1, courbe C3) est moins performant qu'en mélange avec le Gélucire^{®}44/14.

### Exemple 2.3 : ibuprofène

Un autre actif, l'ibuprofène, a été formulé avec les mélanges Gélucire^{®}44/14 / PEG. Le mélange avec actif a été coulé en gélule, de manière similaire aux exemples 2.1 et 2.2 précédents avec le piroxicam et la terfénadine.

La solubilité de l'ibuprofène dans l'eau, à 25°C, est de 21 mg/L.

La dose est de 200 mg, pour un total de 680 mg de formulation.

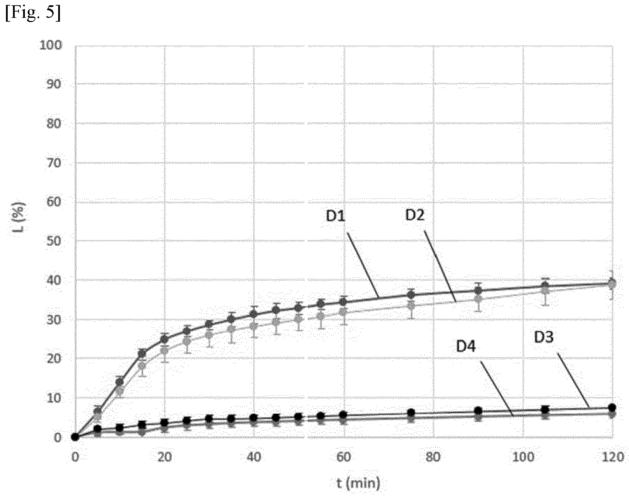

On obtient alors le graphe de la figure 5, illustrant la libération L (%) de l'actif en fonction du temps t en minutes, sur lequel :
- la courbe D1 (invention) correspond à un mélange ibuprofène / Gélucire^{®}44/14 / PEG 6000, dont le ratio massique Gélucire^{®}44/14 / PEG 6000 est de 65 / 35 ;
- la courbe D2 (invention) correspond à un mélange ibuprofène / Gélucire^{®}44/14 / PEG 6000, dont le ratio massique Gélucire^{®}44/14 / PEG 6000 est de 60 / 40 ;
- la courbe D3 (témoin 1) correspond à un mélange ibuprofène / PEG 6000 ;
- la courbe D4 (témoin 2) correspond à de l'ibuprofène seul.

On constate que les mélanges actif (ibuprofène) / Gélucire^{®}44/14 / PEG (courbes D1 et D2) conduisent à une libération d'environ 40% après 120 minutes, ce qui est satisfaisant et correspond à une quantité 5 fois supérieure à celle libérée par le mélange ibuprofène / PEG (témoin 1, courbe D3) ou par l'ibuprofène seul (témoin 2, courbe D4).

Le PEG seul (témoin 1, courbe D3) n'a pas d'impact sur la solubilisation de l'ibuprofène seul (témoin 2, courbe D4) dans le milieu de dissolution.

## Revendications

1. Utilisation comme excipient pharmaceutique ou cosmétique, d'une composition solide à température ambiante sous forme de particules individualisées, ladite composition contenant uniquement :
- macrogolglycéride laurique,
- polyéthylène glycol de masse molaire comprise entre 4000 g/mol et 8000 g/mol.

2. Utilisation selon la revendication 1, **caractérisée en ce que** le PEG présente une masse molaire comprise entre 4000 g/mol et 6000 g/mol.

3. Utilisation selon l'une quelconque des revendications précédentes, **caractérisée en ce que** la composition contient au moins deux PEG de masses molaires différentes, lesdites masses molaires étant comprises entre 4000 g/mol et 8000 g/mol.

4. Utilisation selon l'une quelconque des revendications précédentes, **caractérisée en ce que** le ratio massique macrogolglycéride laurique / polyéthylène glycol est compris entre 40/60 et 70/30.

5. Utilisation selon l'une quelconque des revendications précédentes, **caractérisée en ce que** les particules individualisées se présentent sous la forme de pastilles de taille supérieure ou égale à 1 mm.

6. Utilisation selon l'une quelconque des revendications précédentes, **caractérisée en ce que** la composition est obtenue selon le procédé suivant :
- chauffage du macrogolglycéride laurique et du polyéthylène glycol à une température T1 de manière à obtenir un mélange liquide de macrogolglycéride laurique et de polyéthylène glycol,
- refroidissement du mélange de macrogolglycéride laurique et de polyéthylène glycol à une température T2 inférieure à la température T1, tout en conservant le mélange liquide,
- division du mélange refroidi, avantageusement sous forme de gouttes et gouttelettes,
- refroidissement du mélange divisé, avantageusement sous forme de gouttes et gouttelettes, à une température T3 inférieure à la température T2 pour obtenir des particules solides individualisées.

7. Utilisation selon la revendication 6, **caractérisée en ce que** le macrogolglycéride laurique et le polyéthylène glycol sont mélangés l'un avec l'autre avant l'étape de chauffage, puis le mélange de macrogolglycéride laurique et de polyéthylène glycol est chauffé à la température T1.

8. Utilisation selon la revendication 6 ou la revendication 7, **caractérisée en ce que** la température T1 est supérieure ou égale à 80°C.

9. Utilisation selon l'une quelconque des revendications 6 à 8, **caractérisé en ce que** la température T2 est supérieure ou égale à 45°C, de préférence supérieure ou égale à 55°C, et inférieure ou égale à 60°C.

## Patentansprüche

1. Verwendung einer bei Raumtemperatur festen Zusammensetzung in Form einzelner Partikel als pharmazeutischer oder kosmetischer Hilfsstoff, wobei die Zusammensetzung ausschließlich Folgendes enthält:
- Laurinmakrogolglycerid,
- Polyethylenglykol mit einer Molmasse zwischen 4.000 g/mol und 8.000 g/mol.

2. Verwendung nach Anspruch 1, **dadurch gekennzeichnet, dass** das PEG ein Molekulargewicht zwischen 4.000 g/mol und 6.000 g/mol aufweist.

3. Verwendung nach einem der vorstehenden Ansprüche, **dadurch gekennzeichnet, dass** die Zusammensetzung mindestens zwei PEG mit unterschiedlichen Molmassen enthält, wobei die Molmassen zwischen 4.000 g/mol und 8.000 g/mol liegen.

4. Verwendung nach einem der vorstehenden Ansprüche, **dadurch gekennzeichnet, dass** das Massenverhältnis von Laurinmacrogolglycerid zu Polyethylenglykol zwischen 40/60 und 70/30 liegt.

5. Verwendung nach einem der vorstehenden Ansprüche, **dadurch gekennzeichnet, dass** die einzelnen Partikel in Form von Pastillen mit einer Größe von mindestens 1 mm vorliegen.

6. Verwendung nach einem der vorstehenden Ansprüche, **dadurch gekennzeichnet, dass** die Zusammensetzung nach folgendem Verfahren hergestellt wird:
- Erhitzen von Laurinmacrogolglycerid und Polyethylenglykol auf eine Temperatur T1, um eine flüssige Mischung aus Laurinmacrogolglycerid und Polyethylenglykol zu erhalten,
- Abkühlung der Mischung aus Laurinmacrogolglycerid und Polyethylenglykol auf eine Temperatur T2 unter der Temperatur T1, wobei die Mischung flüssig bleibt,
- Teilung des gekühlten Gemisches, vorzugsweise in Form von Tropfen und Tröpfchen,
- Abkühlung der geteilten Mischung, vorzugsweise in Form von Tropfen und Tröpfchen, auf eine Temperatur T3, die unter der Temperatur T2 liegt, um einzelne Feststoffpartikel zu erhalten.

7. Verwendung nach Anspruch 6, **dadurch gekennzeichnet, dass** das Laurinmacrogolglycerid und das Polyethylenglykol vor der Erhitzungsphase miteinander vermischt werden und die Mischung aus Laurinmacrogolglycerid und Polyethylenglykol anschließend auf die Temperatur T1 erhitzt wird.

8. Verwendung nach Anspruch 6 oder Anspruch 7, **dadurch gekennzeichnet, dass** die Temperatur T1 höher oder gleich 80 °C ist.

9. Verwendung nach einem der Ansprüche 6 bis 8, **dadurch gekennzeichnet, dass** die Temperatur T2 über oder gleich 45 °C, vorzugsweise über oder gleich 55 °C und unter oder gleich 60 °C liegt.

## Claims

1. Use as a pharmaceutical or cosmetic excipient of a composition which is solid at ambient temperature, in the form of individualized particles, said composition containing only :
- lauroyl macrogolglyceride,
- polyethylene glycol having a molar mass comprised between 4000 g/mol and 8000 g/mol.

2. Use according to claim 1, **characterized in that** the PEG has a molar mass comprised between 4000 g/mol and 6000 g/mol.

3. Use according to any one of the preceding claims, **characterized in that** the composition contains at least two PEGs with different molar masses, said molar masses being comprised between 4000 g/mol and 8000 g/mol.

4. Use according to any one of the preceding claims, **characterized in that** the weight ratio of lauroyl macrogolglyceride/polyethylene glycol is comprised between 40/60 and 70/30.

5. Use according to any one of the preceding claims, **characterized in that** the individualized particles are in the form of pellets with a dimension which is greater than or equal to 1 mm.

6. Use according to any one of the preceding claims, **characterized in that** the composition est obtained using the following method:
- heating lauroyl macrogolglyceride and polyethylene glycol to a temperature T1 in a manner such as to obtain a liquid mixture of lauroyl macrogolglyceride and polyethylene glycol,
- cooling the mixture of lauroyl macrogolglyceride and polyethylene glycol to a temperature T2 which is lower than the temperature T1, while keeping the mixture liquid,
- dividing the cooled mixture, advantageously into the form of drops and droplets,
- cooling the divided mixture, advantageously in the form of drops and droplets, to a temperature T3 which is lower than the temperature T2 in order to obtain the individualized solid particles.

7. Use according to claim 6, **characterized in that** lauroyl macrogolglyceride and polyethylene glycol are mixed with each other before heating step, then the mixture of lauroyl macrogolglyceride and polyethylene glycol is heated to the temperature T1.

8. Use according to claim 6 or claim 7, **characterized in that** the temperature T1 is greater than or equal to 80°C.

9. Use according to any one of claims 6 to 8, **characterized in that** the temperature T2 is greater than or equal to 45°C, preferably greater than or equal to 55°C, and less than or equal to 60°C.
